# EUROPÄISCHE PATENTSCHRIFT

(11) **EP 2 759 957 B1**
(45) Veröffentlichungstag und Bekanntmachung des Hinweises auf die Patenterteilung: **31.10.2018**
(21) Anmeldenummer: 13152855.6
(22) Anmeldetag: 28.01.2013
(51) Int. Cl.: G06F 21/62, G06F 19/00

(54) **Übertragungsmittel für sicherheitskritische medizinische Bildinhalte**
Transfer medium for security-critical medical image contents
Moyen de transmission pour contenus médicaux d'images à la sécurité critique

(43) Veröffentlichungstag der Anmeldung: 30.07.2014
(73) Patentinhaber: Siemens Healthcare GmbH, 91052 Erlangen (DE)
(72) Erfinder: Dominick, Lutz, 91330 Eggolsheim (DE); von Stockhausen, Hans-Martin, 91058 Erlangen (DE)

(56) Entgegenhaltungen:
- US-A1- 2008 301 805
- US-A1- 2010 313 274
- US-B1- 7 950 066
- "Digital Imaging and Communications in Medicine; Part 15: Security and System Management Profiles", , 1. Januar 2011 (2011-01-01), Seiten 1-133, XP055067393, Gefunden im Internet: URL:http://medical.nema.org/Dicom/2011/11_ 15pu.pdf [gefunden am 2013-06-19]

## Beschreibung

Die vorliegende Erfindung liegt auf den Gebieten der Medizintechnik und der Informationstechnologie und betrifft insbesondere ein Verfahren, um sicherheitskritische Bildinhalte zu kopieren und möglicherweise in derselben oder in einer anderen Applikation, die auch auf einem anderen Anwendungsgerät implementiert sein kann, einzufügen.

Insbesondere im medizinischen Kontext erfolgt die Bearbeitung zunehmend über Datenverarbeitungseinrichtungen und computerbasiert. Beispielsweise stehen zur Durchführung von chirurgischen, robotergesteuerten Eingriffen, zur Befundung, zur Datenarchivierung etc. eine Vielzahl von unterschiedlichen Applikationen zur Verfügung. Im Bereich der bildgebenden Medizin müssen die akquirierten Bilddaten verarbeitet und abgelegt werden. Dazu ist es hilfreich, einzelne Bildinhalte oder gesamte Bilder aus einer Applikation zu kopieren und an anderer Stelle wieder einzufügen.

Im Stand der Technik wird dazu auf Kurzzeitspeicher, sogenannte Clipboards, zurückgegriffen, in denen zu transferierender Inhalt abgelegt werden kann, um später darauf zugreifen zu können. Die Clipboard-Funktionalität kann somit zum Ausführen von Copy-und-Paste-Befehlen (kopieren und einfügen) verwendet werden. Wie z.B. in der US-A-5,964,834 beschrieben kann dabei jeweils ein Clipboard einem Prozessor eines drahtgebundenen Computernetzwerkes zugeordnet sein.

Im klinischen Kontext werden zunehmend auch mobile Anwendungsgeräte (Laptops, PDA'a, mobile Sensorgeräte, wie Blutdruck/zucker-Messgeräte etc.) eingesetzt. Auch für mobile Anwendungsgeräte soll die Copy-und-Paste-Funktionalität zur Verfügung stehen.

Des Weiteren ist zu berücksichtigen, dass die medizinischen Bilddatensätze in der Regel sicherheitskritischen Inhalt umfassen, der zugriffsgeschützt zu verarbeiten und zu behandeln ist. So muss sichergestellt sein, dass Patienten-identifizierende Angaben (z.B. Name, Alter, Geschlecht, Wohnort etc.) nicht in Kombination mit Bilddaten oder Bilddatenausschnitten über die Grenzen der jeweiligen zugriffsgeschützten Umgebung hinaus gelangen. Diese zugriffsgeschützten Inhalte werden als "protected health information (PHI)" bezeichnet.

Aus sicherheitstechnischen Gründen ist es deshalb nicht möglich, die z.B. von Textverarbeitungsprogrammen bekannte, übliche und hilfreiche Copy-and-Paste-Funktionalität auch im klinisch-medizinischen Bereich zur Verfügung stellen, da mit diesen Techniken die medizinischen Datenschutzkriterien nicht eingehalten werden können.

Im Stand der Technik war es deshalb üblich, die Bildinhalte zu anonymisieren und die generierten anonymisierten Bilddatensätze zu speichern und auch applikationsextern oder workflow-extern verfügbar zu machen.

So offenbart Dokument US 2010/0313274 A1 einen Bilddatenserver, Verfahren, Softwareanwendungen und ein computerlesbares Medium, zum Erhalten von Bildern mit verschiedenen Niveaus von Patientenanonymität durch mehrere Bild-Vertraulichkeits-Softwareschnittstellen. In einem solchen Bilddatenserver werden zumindest zwei Bild-Vertraulichkeits-Softwareschnittstellen bereitgestellt, wobei jeder der Bild-Vertraulichkeits-Softwareschnittstellen eingerichtet ist, Bilder mit einem vorbestimmten Niveau von Patientenvertraulichkeit bereitzustellen, und wobei das Niveau von Patientenvertraulichkeit verschieden ist für jede der Bild-Vertraulichkeits-Softwareschnittstellen.

Dem Dokument "Digital Imaging and Communications in Medicine (DICOM); Part 15: Security and System Management Profiles" ist zu entnehmen, dass der DICOM Standard Verfahren zum Anonymisieren von Bilddaten vorsieht.

Nicht bekannt ist es bislang, eine Copy-and-Paste-Funktionalität für PHI-Datensätze bereitzustellen, die die Sicherheitskriterien erfüllt.

Des Weiteren sollte ein Transfer von Bildinhalten auch über Maschinengrenzen und Applikationsgrenzen hinaus möglich sein.

Die vorliegende Erfindung hat sich deshalb zur Aufgabe gestellt, Mittel zur Umsetzung einer Copy-and-Paste-Funktionalität bereitzustellen, die es erlaubt PHI-Inhalte auch über Workflow- und/oder Maschinengrenzen hinaus zu speichern und zugreifbar zu machen, so dass die Sicherheitskriterien für den medizinischen Bereich erfüllt werden.

Diese Aufgabe wird durch die beiliegenden nebengeordneten Ansprüche gelöst, insbesondere durch ein Computer-implemen tiertes Verfahren, durch ein computer-basiertes Übertragungssystem und durch ein Computerprogrammprodukt.

Nachstehend wird die Lösung der Aufgabe in Bezug auf das beanspruchte Verfahren beschrieben. Hierbei erwähnte Merkmale, Vorteile und/oder alternative Ausführungsformen sind ebenso auch auf die anderen beanspruchten Gegenstände zu übertragen und umgekehrt. Mit anderen Worten können auch das Übertragungssystem oder das Computerprogrammprodukt mit den Merkmalen weitergebildet sein, die im Zusammenhang mit dem Verfahren beschrieben oder beansprucht sind. Die entsprechenden funktionalen Merkmale des Verfahrens werden dabei durch entsprechende gegenständliche Computer-implementierte Module, insbesondere Mikroprozessormodule des Systems ausgebildet. Das Übertragungssystem und das Verfahren können auch als eingebettete Systeme (embedded system) in die Akquisitionsanlage oder in ein medizinisches Gerät (z.B. in ein bildgebendes Akquisitionsgerät, MRT, CT, US, PET etc.) integriert sein.

Gemäß einem Aspekt der Erfindung bezieht sich die Aufgabenlösung auf ein computer-basiertes Verfahren zum Bereitstellen einer Übertragungsfunktion für sicherheitskritische medizinische Datensätze, insbesondere Bilddatensätze, mit PHI-Abschnitten von einer Quellapplikation in eine Zielapplikation, wobei das Verfahren zugleich als sicher erkannte und als unsicher erkannte Zielapplikationen bedienen kann und wobei das Verfahren auf eine PKI-Infrastruktur zugreift, die für jeden Anwender einen Anwender-spezifischen Schlüssel bereitstellt, mit folgenden Verfahrensschritten:
- Nach Erfassen eines Kopierbefehls für zumindest einen Bilddatensatz in der Quellapplikation: Erzeugen eines anonymisierten Bilddatensatzes und Weiterleiten des Bilddatensatzes und des anonymisierten Bilddatensatzes an ein Tradermodul
- Erfassen des jeweiligen Schlüssels und Erstellen von zumindest zwei Datenkonvoluten zu dem Bilddatensatz, umfassend den anonymisierten Bilddatensatz und einen verschlüsselten Bilddatensatz, wobei der verschlüsselte Bilddatensatz entsteht, indem die Bilddaten mit dem erfassten Schlüssel verschlüsselt werden und somit chiffriert vorliegen
- Weiterleiten zumindest des anonymisierten Bilddatensatzes an ein Clipboard und zumindest des verschlüsselten Bilddatensatzes an ein Speichermodul zur Speicherung einer Zuordnungsrelation von einem Schlüssel und zumindest einem dem jeweiligen Schlüssel zugeordneten Bilddatensatz des Datenkonvolutes
- Nach Erfassen eines Einfügebefehls für zumindest einen Bilddatensatz in der Zielapplikation: Erfassen des Schlüssels und automatisches Erfassen, ob die Zielapplikation als gesicherte Applikation registriert ist oder eine unsichere Applikation ist
- Falls die Zielapplikation eine unsichere Applikation ist: Zugriff der Zielapplikation auf das Clipboard, um auf den dem erfassten Schlüssel zugeordneten anonymisierten Bilddatensatz zuzugreifen und auf der Zielapplikation dazustellen
- Falls die Zielapplikation eine sichere Applikation ist: Weiterleiten des Einfügebefehls der Zielapplikation an den Trader, wobei der Trader mit dem erfassten Schlüssel auf das Speichermodul zugreift, um auf den dem jeweiligen Schlüssel zugeordneten verschlüsselten Bilddatensatz zuzugreifen und diesen mit dem Schlüssel zu entschlüsseln und den entschlüsselten Bilddatensatz auf der Zielapplikation bereitzustellen.

Im Folgenden werden die im Rahmen dieser Anmeldung verwendeten Begrifflichkeiten näher erläutert.

Der Begriff "Übertragungsfunktion" umfasst eine Copy-and-Paste-Funktionalität für zu schützende oder sicherheitskritische Inhalte, insbesondere Bildinhalte. Die zu übertragenden Daten können neben Bildinhalten jedoch auch noch andere Datentypen umfassen, wie z.B. Textdaten (Metadaten zu den Bilddaten, beispielsweise die Identität des Patienten betreffend oder Zusatzdaten zu dem Akquisitionsgerät, den Zeitpunkt der Bildakquisition etc). Die Übertragungsfunktion soll nicht auf eine bestimmte Applikation, Maschine oder einen medizinischen Workflow beschränkt sein, sondern auch applikations-, maschinengrenzen-, workflow-übergreifend ausführbar sein, unter Einhaltung der Sicherheitskriterien. Des Weiteren soll die Copy-and-Paste-Funktionalität anwendbar sein, unabhängig davon, ob die Zielapplikation, auf der die zu transferierenden Bildinhalte eingefügt werden sollen, eine sichere oder eine unsichere Applikation ist. Die Übertragungsfunktion wird erfindungsgemäß von einer separaten Instanz als zusätzliches Betriebssystem-Modul oder Add-On bereitgestellt. Sie ist vorzugsweise betriebssystem-spezifisch. Ein wesentlicher Vorteil der erfindungsgemäßen Übertragungsfunktion ist, dass sie herstellerübergreifend einsetzbar ist und somit auch für Quell- und/oder Zielapplikationen anwendbar ist, die von unterschiedlichen Herstellern stammen.

Die PKI-Infrastruktur (public key infrastructure) ist eine Instanz, die Mittel zur Ver- und Entschlüsselung bereitstellt und diese verwaltet. In der Regel umfasst diese eine Zuordnung von (öffentlichen) Schlüsseln zu eineindeutigen Anwender-Identitäten. Sie kann deshalb auch als Zertifizierungs- oder Registrierungsinstanz ausgebildet sein.

Bei den Bilddaten handelt es sich um medizinische Datensätze, insbesondere um Bilddaten, die mit bildgebenden Geräten erfasst werden (Magnetresonanztomographen, Computertomographen, Ultraschallgeräten, Positronen-Emissions-Tomographen etc.). In der medizinischen Bildverarbeitung wird zur Datenübertragung häufig das DICOM Protokoll (Digital Information and Communications in Medicine) eingesetzt. Deshalb liegen die Bilddaten vorzugsweise in einem DICOM-Format vor. Sie umfassen neben den Bildinformationen auch Patienten-identifizierenden Informationen (Name, Identität (z.B. eineindeutige Personalnummer etc.), Alter, Geschlecht, Zusatzinformationen (wie z.B. Anamnesedaten, Diagnosen)). Die Bilddaten umfassen somit PHI-Daten, die nicht aus der sicheren Umgebung hinaus gelangen dürfen. In einer alternativen Anwendung kann es sich bei den Bilddaten jedoch auch um PHI-Datensätze handeln, die im eigentlichen Sinn keine Bilddaten sind, sondern möglicherweise diesen nur zugeordnet sein können (z.B. medizinische Messwerte, Befundungstext etc.), wie Textdaten oder Daten in anderen Formaten.

Die anonymisierten Bilddaten kennzeichnen sich dadurch, dass alle den Patienten identifizierenden Abschnitte oder Anteile manipuliert oder aus dem Datensatz entfernt worden sind. Die anonymisierten Bilddaten können auch mit unterschiedlichen Maßnahmen de-identifiziert werden und enthalten dann keine (den Patienten) identifizierenden Hinweise mehr. Dazu können gezielt und dezidiert vorbestimmbare Kenngrößen aus dem Datensatz eliminiert, generalisiert, ersetzt oder anderweitig manipuliert werden, wie z.B. Name, geographische Daten, Kontaktnummern oder Adressen, darunter auch Email-Adressen, Sicherheitsnummern, sonstige ID-Nummern (z.B. von Gesundheitskarten oder Bankkarten etc.), biometrische Daten, Fotos etc. Eine alternative Maßnahme besteht in der Anwendung von statistischen Verfahren, die darauf basieren, die statistische Wahrscheinlichkeit zu prüfen, ob aus dem de-identifizierten Datensatz wieder die Originaldaten herleitbar sind. Alternativ kann hier auch eine Pseudonymisierung der PHI-Daten eingesetzt werden, die in der Regel eine zentrale Zuordnungsinstanz zwischen Original PHI-Daten und Pseudonym vorhält. Die anonymisierten Bilddaten liegen dabei sichtbar und für jeden lesbar (als Plaintext)- also unverschlüsselt - vor.

Im Unterschied zu den Bilddaten und zu den anonymisierten Bilddaten kennzeichnen sich die verschlüsselten Bilddaten dadurch, dass der Bildinhalt nicht mehr als Plaintext in lesbarer Form vorliegt, sondern verschlüsselt ist und nur mit einem entsprechenden Schlüssel entschlüsselt werden kann. Selbst wenn ein Angreifer in Besitz der verschlüsselten Bilddaten gelangt, kann er daraus ohne den Schlüssel nicht die Bilddaten herleiten.

Der Schlüssel ist vorzugsweise Benutzer-spezifisch und kann entweder in einer zentralen PKI-Instanz oder im Gerät selbst hinterlegt sein. In einer Weiterbildung der Erfindung ist der PKI-Schlüssel gerätespezifisch. Dann bleibt das Verfahren auf das jeweilige Gerät beschränkt. Damit kann eine höhere Sicherheitsstufe eingehalten werden. Vorzugsweise wird ein symmetrisches kryptographisches Verfahren angewendet, so dass der Schlüssel zum Verschlüsseln und zum Entschlüsseln übereinstimmt.

Das Clipboard ist ein Speicher, in dem die zu übertragenden Datensätze vorübergehend abgelegt (gepuffert) werden, so dass die Zielapplikation bei Bedarf darauf zugreifen kann, um diese an einer zu selektierenden Stelle einzufügen. Das Clipboard kann lokal, also Geräte- und Betriebssystem-spezifisch, bereitgestellt werden, so dass die Quellapplikation über ein Clipboard (z.B. Microsoft Windows) und die Zielapplikation über ein Pendant dazu (möglicherweise unterschiedlich zum Clipboard), ein sogenanntes Pasteboard (z.B. Apple iPad), verfügt. Vorzugsweise ist das Clipboard gerätespezifisch, so dass für mehrere Applikationen, die auf einem Gerät installiert sind, ein Clipboard bereitgestellt wird. Bevorzugt ist ein Single-User-Deployment, bei dem zu einer Zeit nur ein User pro Gerät bzw. Device aktiv arbeitet. Dabei können beliebig viele User gleichzeitig am Verfahren mit ihren Devices teilnehmen. In einer weiteren, bevorzugten Ausführungsform der Erfindung wird ein Token verwendet, um die gepufferten Datensätze zu identifizieren bzw. adressierbar zu machen. Damit kann auch ein Multi-Paste-Befehl ausgeführt werden, bei dem mehrere Datensätze in dem Clipboard zwischengespeichert werden können und bei dem die Zielapplikation dann bei Eingabe des Paste-Befehls aus der Menge der gepufferten Datensätze den jeweils relevanten einzufügenden Datensatz auswählen kann. Damit kann die Copy-und-Paste-Funktionalität noch erweitert und flexibler gestaltet werden.

Bei dem Tradermodul handelt es sich um eine Infrastrukturkomponente, die vorzugsweise als ausführbare Datei bzw. Applikation vorgehalten werden kann. Instanzen des Tradermoduls oder mit dem Tradermodul interagierende Module (z.B. in Form von Makros) können auf der Quell- und/oder Zielapplikation ausgebildet sein. Diese Instanzen oder Module können somit teilweise auf der Zielapplikation und teilweise auf der Quellapplikation ausgeführt werden. Das Tradermodul ist vorzugsweise als entfernter Dienst bzw. als Server- oder Cloud-basierte Applikation implementiert, der bzw. die über ein Netzwerk (typischerweise über das Internet) zugreifbar ist. Das Tradermodul kann jedoch auch kliniknetzwerk-intern bereitgestellt werden. Bevorzugt wird ein Tradermodul für jeweils ein registriertes Gerät bereitgestellt. "Registriert" meint in diesem Zusammenhang, dass das Gerät, auf dem die Zielapplikation installiert ist, eine sichere Applikation ist und Zugang zu einem Tradermodul hat (im Unterschied zur unsicheren Zielapplikation, die direkt auf das lokale Clipboard zugreift und nicht auf den Trader). Insbesondere soll ein Tradermodul für Quell- und Zielapplikation implementiert sein. Alternativ können auch Anwendungszenarien bedient werden, bei dem ein Tradermodul für jeweils eine Applikation bereitgestellt wird.

Bei dem Speichermodul handelt es sich um eine Infrastrukturkomponente, die zur Zwischenspeicherung (Pufferung, auch mehrfach sequentiell) von zu übertragenden Daten dient. Vorzugsweise ist das Speichermodul über ein Netzwerk (z.B. cloud-hosted) von den unterschiedlichen Geräten, auf denen die Quell- und/oder Zielapplikation installiert ist, zugreifbar.

Sichere oder unsichere Zielapplikation:
Gemäß einem Aspekt der Erfindung sind zwei Klassen von Applikationen vorgesehen: Sichere und unsichere Zielapplikationen. Eine "sichere" Zielapplikation ist eine Applikation, in der die PHI-Inhalte eingefügt werden sollen, die sich in derselben Sicherheitsumgebung befindet, wie die Quellapplikation und somit keine zusätzlichen Sicherungsmaßnahmen für den Datentransfer erfordert. Die sichere Applikation hat einen Anschluss an das Tradermodul und "weiß" von sich, dass sie "sicher" ist. Die "unsichere" Zielapplikation ist eine Applikation, in der ebenfalls PHI-Inhalte eingefügt werden sollen, die sich jedoch nicht in derselben Sicherheitsumgebung befindet, wie die Quellapplikation, so dass automatisch (also ohne Benutzerinteraktion) zusätzliche Sicherungsmaßnahmen für den Datentransfer erforderlich sind. Die unsichere Applikation verfügt nicht über einen Anschluss an das Tradermodul und kennt auch nicht die Unterscheidung von "sicher" und "unsicher". Gemäß einem Aspekt der Erfindung wird automatisch erfasst, welcher der beiden Klassen die Zielapplikation angehört. Eine sichere Zielapplikation kennzeichnet sich somit unter anderem dadurch, dass sie eine Art Servicekanal, also eine sichere Anbindung an das Tradermodul (im Folgenden auch kurz Trader genannt) hat, während eine unsichere Applikation (wie z.B. Microsoft/Word) dies nicht hat.

In einer vorteilhaften Weiterbildung der Erfindung wird nicht nur erfasst, ob die Applikation (Zielapplikation) sicher bzw. unsicher ist, sondern auch, ob das Gerät, auf dem die jeweilige Applikation (Zielapplikation) implementiert ist, sicher bzw. unsicher ist. Ein sicheres Gerät ist dann ein solches, auf dem ein Tradermodul aktiv ist, der sich von einer entfernten Netzwerkadresse (remote) mit dem Speichermodul verbunden (beispielsweise mit einer url oder einem anderen Link) und sich dort registriert hat.

Grundsätzlich kann das erfindungsgemäße Verfahren innerhalb oder außerhalb eines automatisierten, medizinischen Workflows eingesetzt werden, so dass Copy-and-Paste-Operationen auch über das Clipboard abgewickelt werden können. Damit können ausgewählte Workflow-Schritte sicherer gemacht werden. Beim Erstellen eines klinischen Diagnose-Berichts sollen in einer unsicheren Umgebung zum Beispiel die Elemente der Patienten-Identification (Name, Geburtsdatum, Geschlecht, ID-Nummer im Krankenhaus) oder die Bilder des Patienten von einer Applikation in den Bericht per Clipboard-Copy-and-Paste automatisch übertragen werden. In einer unsicheren Umgebung könnte ein sicherheitskritischer Zugriff auf das Clipboard diese PHI-Daten aus dem Clipboard von der Seite abgreifen.

Im Unterschied zu Makro-Recordern aus dem Stand der Technik, die innerhalb der Applikation den Fluss der ausgeführten Befehle mitschneiden, sind bei dieser Erfindung - insbesondere aus Sicherheitsgründen - alle Befehlsreihenfolgen vorgegeben. Dennoch wird es möglich, Inhalte von einer Applikation auf eine andere (auch rechnerübergreifend) zu transferieren. Ein Copy-and-Paste-Befehl zielt immer auf die gerade selektierten Daten: Bei "copy" sind Daten in der Applikation selektiert, bei "paste" sind die Daten aus dem Clipboard ,selektiert'. Die funktionale Ausführung des Datentransports in die Applikation oder aus ihr heraus erfolgt dann mit üblichen Befehlen, wie z.b. load, store, save, die generisch (applikationsunabhängig) von einem Framework innerhalb der Applikation bereitgestellt werden. Ein Copy-and-Paste-Befehl kann damit sicher und absolut situations- und datenspezifisch ausgeführt werden

Grundsätzlich sind unterschiedliche Deployments des erfindungsgemäßen Verfahrens möglich. Bevorzugt wird ein Single-User-Deployment, bei dem zu einer bestimmten Zeitphase ein User auf einem Gerät eingeloggt ist.

Das erfindungsgemäße Übertragungsverfahren stellt somit sicher, dass die zu transferierenden PHI-Daten von einer Quellapplikation stets unter Einhaltung der Sicherheitsbestimmungen über die Applikationsgrenzen hinaus in die Zielapplikation übertragen werden können.

Falls die Zielapplikation eine unsichere Applikation ist, greift die Zielapplikation - sozusagen: wie gewohnt - mit dem erfassten Schlüssel auf das lokale Clip- (bzw. Pasteboard) zu und erhält den anonymisierten Bilddatensatz, der dem Schlüssel zugeordnet ist. Somit werden nur anonymisierte Datensätze auf dem Zielgerät dargestellt (als Default-Einstellung). Es werden somit automatisch nur die anonymisierten Bilddaten eingefügt. Die Zielapplikation kann in diesem Fall den erfassten Schlüssel nicht auswerten und zur Entschlüsselung verwenden, so dass nur anonymisierte (oder verschlüsselte) Datensätze auf der Zielapplikation eingefügt werden können.

Falls die Zielapplikation eine sichere Applikation ist, ist das Tradermodul aktiviert. Das Tradermodul greift mit dem erfassten Schlüssel automatisch auf die verschlüsselten PHI-Daten zu, die dem Schlüssel jeweils zugeordnet sind, um diese zu entschlüsseln und als Klartext (unverschlüsselt) in der Zielapplikation einzufügen.

Der übliche Ablauf sei im Folgenden kurz beschrieben:
Der User hat Patientendaten in einer medizinischen Applikation geladen und wählt z.B. eines der oder auch mehrere Patientenbilder aus und führt die Funktion ,Clipboard Copy' aus. Die medizinische Applikation legt jedes Bild jetzt zweimal ab, sowohl das verschlüsselte Originalbild, als auch eine (vorzugsweise on-the-fly erstellte) anonymisierte Version desselben Bildes, das keine Patienten-Identität mehr beinhaltet. Die Verschlüsselung erfolgt mit dem Schlüssel des Users aus der bereitgestellten PKI-Infrastruktur. Der User ist über diesen Schlüssel auf jedem Gerät bzw. Device eindeutig identifizierbar. Das verschlüsselte Original-Bild ist nur für diesen User und nur in sicheren Ziel-Applikationen auf diesen Devices wieder lesbar. Deshalb erfolgt die Ablage beider Bilder mit dem User Schlüssel, der auch als primärer Suchschlüssel dient, um die Bilder dieses User's auf jedem Device zu finden. Das Token pro Bild erlaubt dem User, mehrere Bilder gleichzeitig zu kopieren, und bei dem Einfüge- oder Paste-Befehl beliebige Bilder aus der Mehrzahl der abgelegten (zwischen-gespeicherten) Bilder auszuwählen. Die Ablage der Bild-Paare erfolgt mehrfach. Auf dem Clipboard der lokalen Maschine und auf einem sicheren Repository (das Cloud-basiert sein kann), nämlich in dem Speichermodul. Alle Devices, auf denen der User einloggt ist, verbinden sich oder haben sich bereits mit dem Cloud Repository verbunden und werden notifiziert, wenn auf einem dieser Devices für diesen User Bild-Paare in dem Repository und/oder in dem Clipboard abgelegt werden. Alle Devices füllen aufgrund dieser Notifizierung automatisch das lokale Clipboard mit den neuen Bild-Paaren (wobei die Datensätze mit dem Schlüssel und einem Token abgelegt werden). Alle Clipboards auf allen Devices haben dann die gleichen Daten.

Wechselt der User nun zu einer unsicheren Applikation auf einem anderen oder dem gleichen Device und führt einen Paste-Befehl aus, dann greift die unsichere Applikation - wie gewohnt - auf das Clipboard zu, überliest ggf. sogar den Schlüssel und die Tokens (weil sie üblicherweise beide nicht auswerten kann) und wird im einfachsten Fall nur das erste anonymisierte Bild einfügen.

Wechselt der User zu einer sicheren Applikation auf einem anderen oder dem gleichen Device und führt einen Paste-Befehl aus, dann liest die sichere Applikation den User-Schlüssel auf der lokalen Maschine, greift damit auf das Clipboard zu, findet den Schlüssel und die Tokens, und sucht mit dem Schlüssel im Clipboard und in dem Speichermodul bzw. in dem Cloud Repository mit diesem Schlüssel nach Daten, um die so gefunden Daten zu entschlüsseln und dem User alle Original-Bilder zur Auswahl anzuzeigen. Der User wählt Bilder aus und die Applikation fügt die ausgewählten Original-Bilder ein (z.B. in eine sichere klinische Befundungsapplikation). Als Sicherheitsmaßnahme werden Clipboard und Cloud-Repository zeitgesteuert gelöscht (z.B. mittels Speicherlöschoperationen, sogenannten garbage collection mechanisms).

Wesentlich ist, dass eine automatische Erzeugung und Ablage des Datenkonvolutes (wie oben beschrieben: des Daten-Paares mit und ohne PHI-Daten) in der unsicheren Copy-and-Paste-Umgebung durch die zusätzlichen neuen Komponenten in den sicheren Applikationen (diese Komponenten anonymisieren die Daten übergeben die Daten mit und ohne PHI an den SecuSnapTrader) .

Ein wichtige Aspekt ist auch darin zu sehen, dass diese medizinischen Daten auf sowohl alle lokalen Clipboards als auch das Speichermodul bzw. das Cloud-Repository mittels der zusätzlichen und autonomen Infrastruktur-Komponenten dem Tradermodul (auch SecuSnapTrader genannt) und dem Speichermodul (auch genannt: SecuSnapStore) kopiert und verteilt werden. Damit wird ein geräteübergreifendes Copy-and-Paste ermöglichte, und zwar unabhängig vom Typ der Hardware oder vom Typ des Betriebssystems (und ohne zusätzliche Angaben des Users z.B. über das Zielgerät). Vorteilhaft ist, dass sowohl sichere als auch unsichere Applikationen automatisch unterstützt werden. Dies erfolgt transparent für den User und technisch transparent für beliebige unsichere Applikationen.

Gemäß einer bevorzugten Ausführungsform der Erfindung ist ein Token als Identifizierungsmerkmal jeweils einer Instanz eines Bilddatensatzes zugeordnet, so dass auch mehrere Bilddatensätze oder Teile davon in dem Clipboard und/oder in dem Speichermodul akkumuliert und gesammelt werden können und so dass auch ein Multi-Paste-Befehl ausführbar ist. Mit anderen Worten kann ein Anwender auch mehrmals Paste-Befehle hintereinander ausführen und jedes Mal aus den verfügbaren Bilddatensätzen die relevanten auswählen. Das Token dient somit als Zugriffsmittel und Indikator und ist letztendlich für den User gedacht ist, so dass die Gesamtheit der Tokens eine Art "strukturiertes Inhaltsverzeichnis" der verfügbaren Bilddatensätze im Pasteboard und/oder Speichermodul bilden. Damit können auch sequentiell abgelegte (kopierte) PHI-Inhalte ausgewählt oder gemeinsam in der Zielapplikation "gepasted" werden.

Gemäß einer weiter bevorzugten Ausführungsform der Erfindung dient der Schlüssel zugleich zwei unterschiedlichen Funktionen: Zum Einen zum Verschlüsseln des Bilddatensatzes und zum Anderen zum Identifizieren eines Bilddatensatzes bzw. als primärer Suchschlüssel in einer Datenstruktur für die Bilddatensätze (z.B. in dem Clip-/oder Pasteboard oder in dem Speichermodul).

Gemäß einer weiter bevorzugten Ausführungsform der Erfindung wird auf dem Speichermodul und/oder auf dem Clipboard eine Zuordnungsinstanz abgelegt wird, zumindest umfassend den Schlüssel, den dem jeweiligen Schlüssel zugeordneten anonymisierten Bilddatensatz und den dem jeweiligen Schlüssel zugeordneten verschlüsselten Bilddatensatz. Die PHI-Daten im Klartext (plain) werden nicht auf dem Clipboard und auf dem Speichermodul abgelegt, so dass sichergestellt werden kann, dass die zu schützenden PHI-Daten nicht die jeweilige (als sicher verifizierte) Maschinengrenze verlassen.

Gemäß einer weiter bevorzugten Ausführungsform der Erfindung ist es vorgesehen, dass alle Geräte, auf denen der Anwender eingeloggt ist, eine Nachricht erhalten, dass Bilddatensätze für den Anwender im Clipboard und/oder im Speichermodul abgelegt wurden und somit verfügbar sind. Diese Nachricht geht von dem Trader an das Speichermodul und an alle anderen Trader auf den anderen Geräten. Die andere Trader auf den anderen Geräten hatten sich nach dem Einloggen des Users für die Benutzung des Speichermoduls beim dem Trader (sozusagen parallel oder neben dem Speichermodul registriert). Damit kann der Trader neben dem Speichermodul in diesem Speichermodul selber die Aufrufadresse der Trader auf den anderen Geräten ablegen und für das Senden von Nachrichten wieder lesen. Dabei wird auch die Aufrufadresse der anderen Trader mit dem Schlüssel als Primärschlüssel in dem Speichermodul vorgehalten. Mit diesem Aspekt der Erfindung wird ein verteilter Clipboard-Mechanismus mit Fernzugriff und Notifizierung und automatischer Verteilung der Clipboard-Daten zur Verfügung gestellt.

Gemäß einer weiter bevorzugten Ausführungsform der Erfindung ist es vorgesehen, dass für jeweils ein Anwendungsgerät auf dem die Quellapplikation und/oder die Zielapplikation implementiert sind, ein Trader installiert wird.

Ein üblicher Ablauf des Übertragungsverfahrens ist so ausgebildet: Erhält ein Trader, der auf einem Gerät installiert ist, eine Nachricht, dass Bilddatensätze für den Anwender im Clipboard und/oder im Speichermodul abgelegt wurden und somit verfügbar sind, liest der Trader die anonymisierten Bilddatensätze aus dem Speichermodul aus und legt diese im lokalen Clipboard auf dem Gerät ab. Eine unsichere Zielapplikation liest die anonymisierten Bilddatensätze genauso, als würden sich die unsichere Zielapplikation und die Quellapplikation auf demselben Gerät befinden.

Als Weiterbildung und Optimierung können alle Bilddaten lokal im Clipboard gehalten werden, um den Zugriff von einer sicheren Zielapplikation auf die Bilddaten zu beschleunigen.

Ein wesentlicher Vorteil des vorstehend beschriebenen Verfahrens ist darin zu sehen, dass mit einer Übertragungsfunktion sowohl sichere als auch unsichere Applikationen gleichermaßen bedient werden können. Des Weiteren kann die Übertragungsfunktion auch über Maschinengrenzen hinaus (cross-device) angewendet werden.

Es liegt ebenso im Rahmen der Erfindung, die vorstehend erwähnten Schritte des Verfahrens nicht zwangsläufig in der vorstehend beschriebenen Reihenfolge zur Ausführung zu bringen. Alternativ ist es beispielsweise auch möglich, zunächst mehrere Kopierbefehle und dann einen oder mehrere Einfügebefehle auf der Zielapplikation auszuführen. In einer weiteren Ausführungsform können die Verfahrensschritte des Kopierens und des Einfügens der Bilddaten ineinander verschachtelt sein (Interleaving), so dass bei Erfassen eines Einfügebefehls unmittelbar ein Fenster auf dem Monitor des Zielsystems erscheint und aktivierbar ist, über das ein oder mehrere der verfügbaren Bilddaten selektiv zum Einfügen auswählbar sind. Dieses Fenster kann entweder für ein Bild oder für eine Gruppe von Bildern (z.B. eine Bildserie einer Untersuchung) - etwa durch einen Mausclick - aktiviert werden.

Darüber hinaus ist es möglich, dass einzelne Abschnitte des vorstehend beschriebenen Verfahrens als einzelne verkaufsfähige Einheiten und restliche Abschnitte des Verfahrens als andere verkaufsfähige Einheiten ausgebildet werden können. Damit kann das erfindungsgemäße Verfahren als verteiltes System auf unterschiedlichen computer-basierten Instanzen (z.B. Client-Server-Instanzen) zur Ausführung kommen. So ist es beispielsweise möglich, dass das Tradermodul seinerseits unterschiedliche Sub-Module umfasst, die teilweise auf der Quellapplikation, teilweise auf der Zielapplikation und/oder teilweise auf anderen computer-basierten Instanzen implementiert sind.

Eine weitere Lösung der vorstehenden Aufgabe bezieht sich auf ein Computerprogrammprodukt gemäß dem beiliegenden Anspruch. Eine weitere Aufgabenlösung sieht ein Computerprogramm vor, das Computerinstruktionen umfasst. Die Computerinstruktionen können unmittelbar auf einem Speicher eines Computers gespeichert sein oder über ein Netzwerk geladen werden und umfassen von dem Computer lesbare Befehle, die zur Ausführung des vorstehend beschriebenen Verfahrens bestimmt sind, wenn die Befehle auf dem Computer ausgeführt werden. Das Computerprogramm kann auch auf einem Speichermedium gespeichert sein oder es kann über ein entsprechendes Netzwerk von einem Server heruntergeladen werden.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:
- Figur 1: Eine Prinzipdarstellung für einen Kopier- und Einfügebefehl,
- Figur 2: Eine schematische Darstellung zur Ausführung eines Kopier- und Einfügebefehls für eine sichere Zielapplikation gemäß einer bevorzugten Ausführungsform der Erfindung
- Figur 3: Eine schematische Darstellung zur Ausführung eines Kopier- und Einfügebefehls für eine unsichere Zielapplikation gemäß einer bevorzugten Ausführungsform der Erfindung
- Figur 4: Ein Ablaufdiagramm gemäß einem bevorzugten Ablauf eines erfindungsgemäßen Verfahrens.

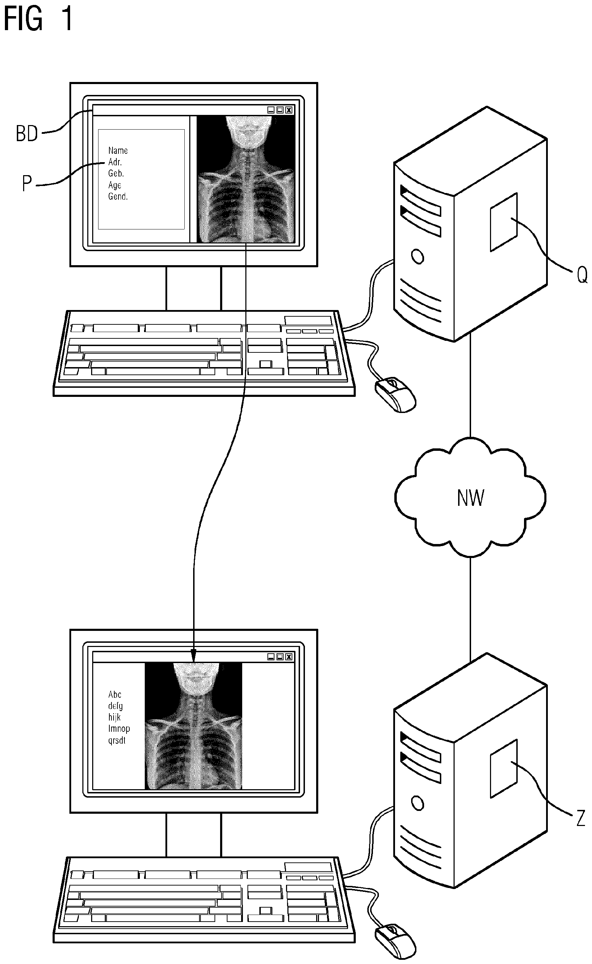

Wie in Figur 1 dargestellt, sollen Bilddaten von einer Quellapplikation Q in eine Zielapplikation Z übertragen werden. Die Quellapplikation Q läuft auf einem ersten Gerät und die Zielapplikation Z auf einem zweiten, wobei die Geräte G über ein Netzwerk in Datenaustausch stehen.

Dies gilt soweit auch für bekannte Copy-and-Paste-Befehle. Im Unterschied zu bekannte Verfahren hebt die Erfindung darauf ab, dass die zu übertragenden Daten Bilddaten BD umfassen und des Weiteren geheimhaltungsbedürftige, zu schützende oder sichernde Daten, so genannte protected health information, kurz: PHI-Daten, die in den Figuren mit dem Bezugszeichen P gekennzeichnet sind. Die PHI-Daten können in unterschiedlichen Formaten vorliegen, vorzugsweise in einem DICOM Format und umfassen in der Regel Bilddaten zu einem Gesundheitszustand eines Patienten (Diagnose, Befunde, Therapie-bezogenen Datensätze, Kostenträger-bezogene Datensätze etc.). Die PHI-Daten P haben stets einen medizinischen Inhalt.

Sollen nun Datensätze mit PHI-Abschnitten P kopiert und an anderer Stelle eingefügt werden, können die bisherigen Copy-and-Paste-Funktionen, die in der Regel vom Betriebssystem bereitgestellt werden nicht verwendet werden, weil die Sicherheitskriterien (Schutz von PHI-Daten) nicht automatisch eingehalten werden können.

Hier setzt die Erfindung an und stellt eine IT-Infrastruktur vor, die automatisch ein sicheres Ausführen eines Copy-and-Paste-Befehls auch über Applikations- oder Gerätegrenzen hinaus ermöglicht.

Grundsätzlich kann das erfindungsgemäß Übertragungsverfahren für Bilddatensätze BD mit PHI-Abschnitte P sowohl sichere als auch unsichere Zielapplikationen bedienen. Eine Zielapplikation gilt als "sicher", wenn es entweder dieselbe Applikation ist, wie die Quellapplikation Q oder sich in derselben informationstechnologischen Umgebung befindet, wie die Quellapplikation Q oder in dem Übertragungssystem registriert ist und somit über einen Anschluss an ein Tradermodul Tr und/oder an ein Speichermodul S verfügt. Das Tradermodul Tr kennt per Konfiguration die Adresse bzw. URL vom Speichermodul S. Beim Start des Tradermoduls Tr greift das Tradermodul Tr über die URL des Speichermoduls S auf das Speichermodul S zu und übergibt diesem seine eigene URL, also die URL des Tradermoduls Tr. Weiterhin übergibt das Tradermodul Tr den Schlüssel key des aktuellen Users an das Speichermodul S. Beim De-Registrieren löscht das Tradermodul Tr seine URL im Speichermodul S. Das Tradermodul Tr kann später auf das Speichermodul S zugreifen, um für einen Schlüssel key Daten zu übermitteln. Dann wird das Speichermodul S diese Daten für alle registrierten anderen Tradermodule Tr übermitteln, wenn diese Tradermodule Tr sich mit demselben Schlüssel key registriert hatten.

Alle anderen Zielapplikationen Z gelten prima facie als "unsicher".

Bei Ausführung des erfindungsgemäßen Übertragungsverfahrens für PHI-Bilddatensätze BD wird nun automatisch erfasst, ob die Zielapplikation Z sicher oder unsicher ist.

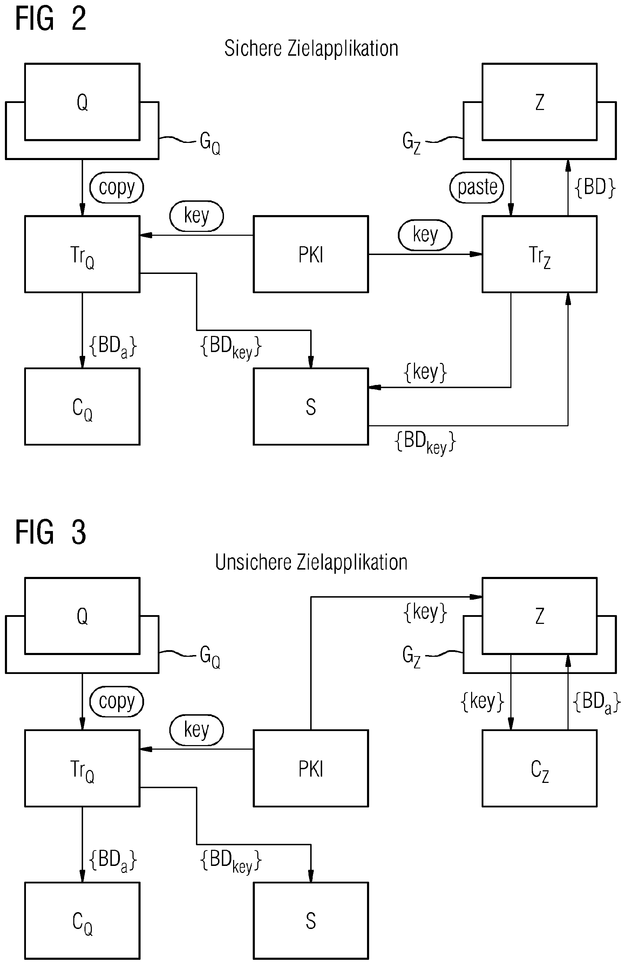

Falls es sich um eine sichere Zielapplikation Z handelt, ist ein Ausführungsbeispiel in Figur 2 dargestellt. Das System hat erfasst, dass die Zielapplikation Z als sicher gilt. Auf der Quellapplikation Quellapplikation Q, die auf einem Gerät G_{Q} ausgeführt wird, wird ein Kopierbefehl erfasst und an ein Tradermodul Tr weitergeleitet, das in diesem Fall der Quellapplikation Q zugeordnet ist und deshalb das Bezugszeichen Tr_{Q} trägt. Die Quellapplikation Q überträgt die zu kopierenden Bilddaten BD ebenfalls an das Tradermodul Tr_{Q}. Daraufhin kann das Tradermodul Tr_{Q} einem dem Gerät G_{Q} oder dem Anwender oder der Applikation Quellapplikation Q zugeordneten Schlüssel key aus einer zentralen PKI-Instanz (public key infrastrucutre) PKI erfassen. Der Schlüssel key dient zur Verschlüsselung des einzufügenden Bilddatensatzes BD. Des Weiteren erstellt das Tr einen anonymisierten Bilddatensatz BDₐ, der keine den Patienten identifizierenden Inhalte mehr umfasst.

Erfindungsgemäß wird also auf Basis der zu übertragenden Bilddaten BD ein Datenkonvolut erstellt, umfassend einen verschlüsselten Bilddatensatz BD_{key} und einen anonymisierten Bilddatensatz BDₐ. Damit entstehen drei Instanzen des originalen Bilddatensatzes BD:
1. der originale Bilddatensatz BD
2. der anonymisierte Bilddatensatz BDₐ und
3. der verschlüsselte Bilddatensatz BD_{key}.

In einer bevorzugten Ausführungsform ist es vorgesehen, dass der anonymisierte Bilddatensatz BDₐ in ein (in der Regel) lokales Clipboard C (in diesem Fall, wie in Fig. 2 dargestellt: C_{Q}, weil das Clipboard der Quellapplikation Q zugeordnet ist) überträgt und dass der verschlüsselte Bilddatensatz BD_{key} in ein Speichermodul S übertragen wird.

Das Speichermodul S kann als Server ausgebildet sein und in einer Netzwerkarchitektur als Cloud-basierter Dienst bereitgestellt werden. Dasselbe gilt für das Tradermodul Tr.

Sobald nun auf der Zielapplikation Z ein Einfügebefehl erfasst wird, wird dieser an das (ebenfalls vorzugsweise lokale) Tradermodul Tr weitergeleitet. Das Tradermodul Tr kann dann von der PKI-Instanz PKI den zugehörigen Schlüssel key auslesen und mit diesem Schlüssel key auf das Speichermodul S zugreifen.

Ein wesentliches Merkmal dieser Erfindung ist nun darin zu sehen, dass der Schlüssel zugleich zwei Funktionen erfüllt:
1. Er dient als Ver- und Entschlüsselungsmittel und
2. Er dient zugleich auch als Primärschlüssel zum Zugriff auf einen bestimmten Datensatz in den Speichern (Speichermodul S, Clipboard C), also als Identifier oder Zugriffsmittel.

Mit dem Schlüssel key findet das Tradermodul Tr (in diesem Fall Tr_{Z}) den für die Zielapplikation bestimmten verschlüsselten Bilddatensatz BD_{key}. Dieser kann dann mit dem Schlüssel key auf dem Tradermodul Tr (oder in der Applikation Z) entschlüsselt und als Klartext (also entschlüsselt) auf der Zielapplikation Z bereitgestellt werden. Vorzugweise enthält der entschlüsselte Bilddatensatz die PHI-Informationen. Es kann voreingestellt sein, dass der entschlüsselte Bilddatensatz mit dem originalen Bilddatensatz BD übereinstimmt. Andernfalls kann eingestellt sein, dass bestimmte Abschnitte nicht mit eingefügt werden oder, dass zusätzliche Abschnitte mit eingefügt werden. In diesem Fall enthält der entschlüsselte Bilddatensatz, der auf der Zielapplikation Z eingefügt wird, Meta-Informationen, z.B. über die Quellapplikation Q, über den Zeitpunkt des Kopierbefehls, den Zeitpunkt des Einfügebefehls und/oder weitere Daten und/oder eine Kennzeichnung (Hervorhebung) der PHI-Daten P.

Falls die Zielapplikation Z als unsichere Applikation ausgewertet wurde, wird der Ablauf des Übertragungsverfahrens unter Bezugnahme auf Fig. 3 erläutert. Die Verfahrensschritte, die im Zusammenhang mit dem Kopierbefehl ausgeführt werden, stimmen mit denjenigen aus dem Szenario der sicheren Applikation (s. oben und auf der linken Seite in Fig. 3 dargestellt) überein. Der Einfügevorgang unterscheidet sich jedoch. Bei Erfassen eines Einfügebefehls auf der Zielapplikation Z, wird der Schlüssel key erfasst, mit dem dann auf das (in der Regel: lokale) Clipboard C (in diesem Fall C_{Z}) zugegriffen wird, um auf den dem Schlüssel key zugeordneten anonymisierten Bilddatensatz BDₐ zuzugreifen und diesen an die Zielapplikation Z zu übertragen bzw. an die vom Cursor bestimmte Position einzufügen.

Wesentlich ist, dass sich das Format der Dateien, auf denen der Kopier- und der Einfügebefehl ausgeführt werden soll, unterscheiden kann. So kann die Quellapplikation Q auf einem DICOM-Format basieren und die Zielapplikation Z auf einem .txt oder .ppt Format oder einem anderen Format, das nicht zwingend für Bildinhalte ausgelegt sein muss.

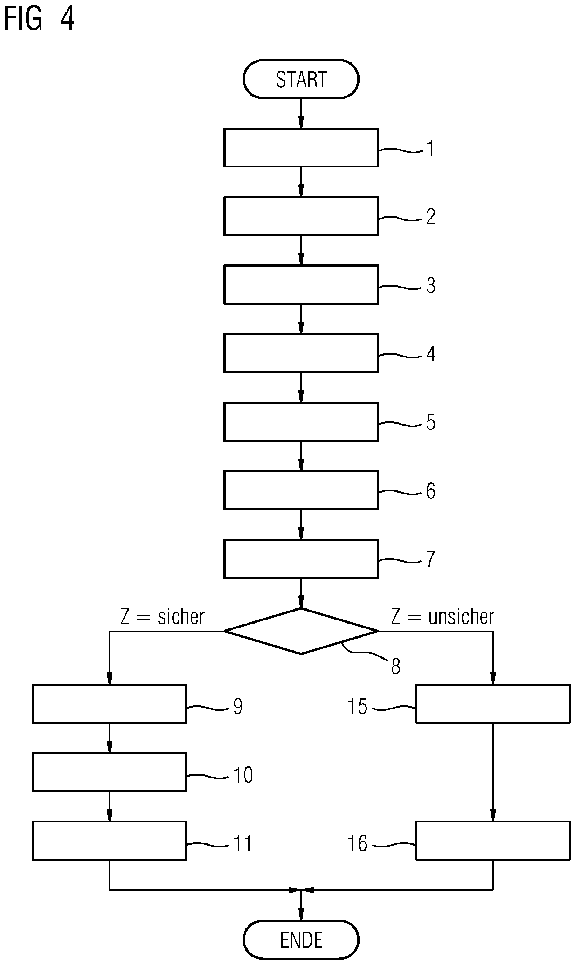

Unter Bezugnahme auf Fig. 4 wird im Folgenden der Ablauf des Übertragungsverfahrens gemäß einer bevorzugten Ausführungsform beschrieben.

Nach dem Start des Verfahrens wird in **Schritt 1** der Kopierbefehle erfasst. Dies erfolgt auf der Quellapplikation Q. Daraufhin wird in **Schritt 2** der Schlüssel key erfasst. Dies kann auf der Quellapplikation Q oder auf dem Tradermodul Tr ausgeführt werden. In **Schritt 3** wird das Datenkonvolut erstellt, umfassend 1. Den anonymisierten Bilddatensatz BDₐ und 2. den verschlüsselten Bilddatensatz BD_{key}. In **Schritt 4** wird zumindest der anonymisierte Bilddatensatz BDₐ an das Clipboard C und in **Schritt 5** der verschlüsselte Bilddatensatz BD_{key} an das Speichermodul S weitergeleitet und dort zwischengespeichert. Es können auch sequentiell mehrere Bilddatensätze in den Speichern C, S abgelegt werden, die über ein Token T identifizierbar sind. Auch ist es möglich das gesamte, generierte Datenkonvolut (mit anonymisiertem Bilddatensatz BDₐ und verschlüsseltem Bilddatensatz BD_{key}) auf dem Clipboard C und/oder auf dem Speichermodul S abzulegen. Die Schritte 3 bis 5 werden bevorzugt auf dem Tradermodul Tr ausgeführt. Es ist jedoch auch möglich, einzelne Verfahrensschritte auf die Quellapplikation Q und/oder andere Instanzen auszulagern. In **Schritt 6** wird ein Einfügebefehl auf der Zielapplikation Z erfasst. In **Schritt 7** wird der Schlüssel key erfasst. In **Schritt 8** wird ausgewertet, ob die Zielapplikation Z vom Typ "sicher" (linksseitig in Fig. 4 dargestellt) oder "unsicher" (rechtsseitig dargestellt) ist.

Falls es sich um eine sichere Zielapplikation Z handelt, wird in **Schritt 9** der Einfügebefehl an den Trader bzw. das Tradermodul Tr weitergeleitet, so dass dieser in **Schritt 10** mit dem erfassten Schlüssel key auf das Speichermodul S und/oder auf das Clipboard C zugreifen kann, um den dem Schlüssel key zugeordneten verschlüsselten Bilddatensatz BD_{key} abzurufen und um diesen in **Schritt 11** ebenfalls mittels des Schlüssels key zu entschlüsseln und um daraufhin das Ergebnis (die originalen Bilddaten BD) in die Zielapplikation Z einzufügen. Daraufhin endet das Verfahren oder kann erneut z.B. für andere Bilddatensätze BD fortgesetzt werden.

Falls es sich um eine unsichere Zielapplikation Z handelt, wird seitens der Zielapplikation Z in **Schritt 15** mit dem erfassten Schlüssel key auf das Speichermodul S zugegriffen, um den anonymisierten Bilddatensatz BDₐ aus dem Clipboard C abzurufen und diese in **Schritt 16** an die Zielapplikation Z zum Zwecke des Einfügens weiterzuleiten. Daraufhin kann das Verfahren ebenfalls enden oder erneut fortgesetzt werden.

Abschließend sei darauf hingewiesen, dass die vorstehende Beschreibung der Erfindung mit den Ausführungsbeispielen grundsätzlich nicht einschränkend im Hinblick auf eine bestimmte physikalische Realisierung der Erfindung zu verstehen ist. So ist es für einen Fachmann insbesondere offensichtlich, dass die Erfindung grundsätzlich nicht auf ein bestimmtes Datenformat (z.B. DICOM Bilddaten) beschränkt ist, sondern ebenso auch für andere Datenformate (Textdaten etc.) verwendet werden kann. Darüber hinaus ist es auch nicht zwingend notwendig, auf eine Cloud-basierte Kommunikationstechnologie zurückzugreifen. Beispielsweise können hier auch proprietäre Protokolle zur Prozesskommunikation verwendet werden. Darüber hinaus kann die Erfindung teilweise oder vollständig in Software und/oder in Hardware implementiert sein. Darüber hinaus kann das erfindungsgemäße Verfahren bzw. das Übertragungssystem auch auf mehrere physikalische Produkte, umfassend Computerprogrammprodukte, verteilt realisiert werden. Somit ist es möglich, einen Teil der Steuerung der Übertragung auf dem Quellsystem Q zu implementieren und einen restlichen Teil der Steuerung auf dem Zielsystem Z und umgekehrt.

## Patentansprüche

1. Verfahren zum Bereitstellen einer Übertragungsfunktion für sicherheitskritische medizinische Bilddatensätze (BD) mit Protected Health Information, PHI-Abschnitten (P) von einer Quellapplikation (Q) in eine Zielapplikation (Z), wobei das Verfahren zugleich als sicher erkannte und als unsicher erkannte Zielapplikationen (Z) bedienen kann und wobei das Verfahren auf eine PKI-Infrastruktur zugreift, die für jeden Anwender einen Anwender-spezifischen Schlüssel (key) bereitstellt, mit folgenden Verfahrensschritten:
- Nach Erfassen eines Kopierbefehls für zumindest einen Bilddatensatz (BD) in der Quellapplikation (Q): Weiterleiten des Bilddatensatzes (BD) und eines daraus erzeugten anonymisierten Bilddatensatzes (BDₐ) an ein Tradermodul (Tr)
- Erfassen des jeweiligen Schlüssels (key) und Erstellen von zumindest zwei Datenkonvoluten zu dem Bilddatensatz (BD), umfassend den anonymisierten Bilddatensatz (BDₐ) und einen verschlüsselten Bilddatensatz (BD_{key})
- Weiterleiten zumindest des anonymisierten Bilddatensatzes (BDₐ) an ein Clipboard (C) und zumindest des verschlüsselten Bilddatensatzes (BD_{key}) an ein Speichermodul (S) zur Speicherung einer Zuordnungsrelation von einem Schlüssel (key) und zumindest einem dem jeweiligen Schlüssel (key) zugeordneten Bilddatensatz des Datenkonvolutes
- Nach Erfassen eines Einfügebefehls für zumindest einen Bilddatensatz (BD) in der Zielapplikation (Z): Erfassen des Schlüssels (key) und automatisches Erfassen, ob die Zielapplikation (Z) als gesicherte Applikation registriert ist oder eine unsichere Applikation ist
- Falls die Zielapplikation (Z) eine unsichere Applikation ist: Zugriff der Zielapplikation (Z) auf das Clipboard (C), um auf den dem erfassten Schlüssel (key) zugeordneten anonymisierten Bilddatensatz (BDₐ) zuzugreifen und auf der Zielapplikation (Z) dazustellen
- Falls die Zielapplikation (Z) eine sichere Applikation ist: Weiterleiten des Einfügebefehls der Zielapplikation (Z) an den Trader (Tr), wobei der Trader (Tr) mit dem erfassten Schlüssel (key) auf das Speichermodul (S) zugreift, um auf den dem jeweiligen Schlüssel zugeordneten verschlüsselten Bilddatensatz (BD_{key}) zuzugreifen und diesen mit dem Schlüssel (key) zu entschlüsseln und den entschlüsselten Bilddatensatz (BD) auf der Zielapplikation (Z) bereitzustellen.

2. Verfahren nach Anspruch 1, bei dem ein Token (T) als Identifizierungsmerkmal jeweils einer Instanz eines Bilddatensatzes (BD, BDₐ, BD_{key}) zugeordnet wird, so dass auch mehrere Bilddatensätze (BD) oder Teile davon in dem Clipboard (C) und/oder in dem Speichermodul (S) gesammelt werden können und so dass auch ein Multi-Paste-Befehl ausführbar ist.

3. Verfahren nach einem der vorstehenden Verfahrensansprüche, bei dem der Schlüssel (key) zugleich für zwei unterschiedliche Funktionen verwendet wird, nämlich zum Verschlüsseln des Bilddatensatzes (BD) und zum Identifizieren eines Bilddatensatzes (BD).

4. Verfahren nach einem der vorstehenden Verfahrensansprüche, bei dem auf dem Speichermodul (S) eine Zuordnungsinstanz abgelegt wird, zumindest umfassend den Schlüssel (key), den dem jeweiligen Schlüssel (key) zugeordneten anonymisierten Bilddatensatz (BDₐ) und den dem jeweiligen Schlüssel (key) zugeordneten verschlüsselten Bilddatensatz (BD_{key}).

5. Verfahren nach einem der vorstehenden Verfahrensansprüche, bei dem alle Geräte (G), auf denen der Anwender eingeloggt ist, eine Nachricht (N) erhalten, dass Bilddatensätze (BD) für den Anwender im Clipboard (C) und/oder im Speichermodul (S) abgelegt wurden.

6. Verfahren nach einem der vorstehenden Verfahrensansprüche, bei dem für jeweils ein Gerät (G) auf dem die Quellapplikation (Q) und/oder die Zielapplikation (Z) implementiert sind, ein Trader (Tr) installiert wird.

7. Computerprogrammprodukt mit Computerprogrammcode zur Durchführung des Verfahrens nach einem der vorstehenden Verfahrensansprüche, wobei der Computerprogrammcode in einen Speicher eines Computers oder eines computer-basierten Gerätes (G) geladen oder ladbar ist, wenn der Computerprogrammcode auf dem Computer ausgeführt wird.

8. Übertragungssystem für sicherheitskritische medizinische Bilddatensätze mit Protected Health Information, PHI-Abschnitten von einer Quellapplikation (Q) an eine Zielapplikation (Z), mit:
- Einer Quellapplikation (Q), in der zumindest ein Kopierbefehl für zumindest einen Bilddatensatz (BD) erfasst wird
- Einer Zielapplikation (Z), in der zumindest ein Einfügebefehl erfasst wird
- Einer PKI-Infrastruktur, die für jeden Anwender einen Anwender-spezifischen Schlüssel (key) bereitstellt
- Einem Tradermodul (Tr), auf dem für jeden Bilddatensatz (BD) ein anonymisierter Bilddatensatz (BDₐ) und ein verschlüsselter Bilddatensatz (BD_{key}) generiert wird
- Einem Speichermodul (S), in dem zumindest der verschlüsselte Bilddatensatz (BD_{key}) abgelegt wird
- Einem Clipboard (C), in dem zumindest der anonymisierte Bilddatensatz (BDₐ) abgelegt wird
- wobei das Übertragungssystem zugleich als sicher erkannte und als unsicher erkannte Zielapplikationen (Z) bedienen kann, wobei bei einer unsicheren Zielapplikationen (Z) diese mit dem Schlüssel (key) auf das Clipboard (C) zugreift, um den dem Schlüssel zugeordneten anonymisierten Bilddatensatz (BDₐ) zu erfassen und auf der Zielapplikationen (Z) darzustellen und wobei bei einer sicheren Zielapplikationen (Z) das Tradermodul (Tr) mit dem Schlüssel (key) auf das Speichermodul (S) zugreift, um den verschlüsselten Bilddatensatz (BD_{key}) zu erfassen und mit dem Schlüssel zu entschlüsseln und den so entschlüsselten Bilddatensatz (BD) auf der Zielapplikation (Z) einzufügen.

## Claims

1. Method for providing a transfer function for security-critical medical image data records (BD) with Protected Health Information, PHI, sections (P) from a source application (Q) to a destination application (Z), wherein the method can simultaneously serve destination applications (Z) that are recognized as secure and destination applications (Z) that are recognized as nonsecure and wherein the method accesses a PKI infrastructure that provides a user-specific key (key) for each user, having the following method steps:
- following detection of a copy command for at least one image data record (BD) in the source application (Q): the image data record (BD) and an anonymized image data record (BDₐ) produced therefrom are forwarded to a trader module (Tr)
- the respective key (key) is detected and at least two data convolutes for the image data record (BD), comprising the anonymized image data record (BDₐ) and an encrypted image data record (BD_{key}), are created
- at least the anonymized image data record (BDₐ) is forwarded to a clipboard (C) and at least the encrypted image data record (BD_{key}) is forwarded to a memory module (S) for storing an association relation for a key (key) and at least one image data record associated with the respective key (key) from the data convolute
- following detection of a paste command for at least one image data record (BD) in the destination application (Z): the key (key) is detected and it is automatically detected whether the destination application (Z) is registered as a secured application or is a nonsecure application
- if the destination application (Z) is a nonsecure application: the destination application (Z) accesses the clipboard (C) in order to access the anonymized image data record (BDₐ) associated with the detected key (key) and to present it on the destination application (Z)
- if the destination application (Z) is a secure application: the paste command from the destination application (Z) is forwarded to the trader (Tr), wherein the trader (Tr) uses the detected key (key) to access the memory module (S) in order to access the encrypted image data record (BD_{key}) associated with the respective key and to decrypt it using the key (key) and to provide the decrypted image data record (BD) on the destination application (Z).

2. Method according to Claim 1, in which a token (T) is associated with a respective entity of an image data record (BD, BDₐ, BD_{key}) as an identification feature, as a result of which a plurality of image data records (BD) or portions thereof can also be gathered on the clipboard (C) and/or in the memory module (S) and as a result of which a multipaste command can also be executed.

3. Method according to either of the preceding method claims, in which the key (key) is simultaneously used for two different functions, namely for encrypting the image data record (BD) and for identifying an image data record (BD) .

4. Method according to one of the preceding method claims, in which the memory module (S) is used to store an association entity, at least comprising the key (key), the anonymized image data record (BDₐ) associated with the respective key (key) and the encrypted image data record (BD_{key}) associated with the respective key (key).

5. Method according to one of the preceding method claims, in which all appliances (G) on which the user is logged in receive a message (N) that image data records (BD) have been stored for the user on the clipboard (C) and/or in the memory module (S).

6. Method according to one of the preceding method claims, in which a trader (Tr) is installed for a respective appliance (G) on which the source application (Q) and/or the destination application (Z) are implemented.

7. Computer program product with computer program code for carrying out the method according to one of the preceding method claims, wherein the computer program code is loaded or can be loaded into a memory of a computer or a computer-based appliance (G) when the computer program code is executed on the computer.

8. Transfer system for security-critical medical image data records with Protected Health Information, PHI, sections from a source application (Q) to a destination application (Z), having:
- a source application (Q) in which at least one copy command for at least one image data record (BD) is detected
- a destination application (Z) in which at least one paste command is detected
- a PKI infrastructure that provides a user-specific key (key) for each user
- a trader module (Tr) on which an anonymized image data record (BDₐ) and an encrypted image data record (BD_{key}) are generated for each image data record (BD)
- a memory module (S) that is used to store at least the encrypted image data record (BD_{key})
- a clipboard (C) that is used to store at least the anonymized image data record (BDₐ)
- wherein the transfer system can simultaneously serve destination applications (Z) that are recognized as secure and destination applications (Z) that are recognized as nonsecure, wherein in the case of a nonsecure destination application (Z) the latter uses the key (key) to access the clipboard (C) in order to detect the anonymized image data record (BDₐ) associated with the key and to present it on the destination application (Z) and wherein in the case of a secure destination application (Z) the trader module (Tr) uses the key (key) to access the memory module (S) in order to detect the encrypted image data record (BD_{key}) and to decrypt it using the key and to paste the thus decrypted image data record (BD) on the destination application (Z).

## Revendications

1. Procédé de mise à disposition d'une fonction de transmission pour des jeux (BD) de données d'image médicales critiques du point de vue de la sécurité avec Protected Health Information,
de parties PHI (P) d'une application (Q) à une application (Z) cible, dans lequel le procédé peut desservir en même temps des applications (Z) cibles considérées comme sûres et considérées comme non sûres et dans lequel le procédé accède à une infrastructure PKI, qui met à disposition de chaque utilisateur une clé (key) spécifique à l'utilisateur, comprenant les stades de procédé suivants :
- après réception d'une instruction de copie d'au moins un jeu (BD) de données d'image dans l'application (Q) source : acheminement du jeu (BD) de données d'image et d'un jeu (BDₐ) de données d'image anonymisé qui en est produit vers un module (Tr) de trader
- détection de la clé (key) respective et établissement d'au moins deux convolutions de données pour le jeu (BD) de données d'image, comprenant le jeu (BDₐ) de données d'image anonymisé et un jeu (BD_{key} ) de données d'image chiffré
- acheminement au moins du jeu (BDₐ) de données d'image anonymisé vers un clipboard (C) et au moins du jeu (BD_{key}) de données d'image chiffré vers un module (S) de mémoire pour mémoriser une relation d'association d'une clé (key) et d'au moins l'un du jeu de données d'image, associé à la clé (key) respective, de la convolution de données
- après détection d'une instruction d'insertion d'au moins un jeu (BD) de données d'image dans l'application (Z) cible : détection de la clé (key) et détection automatique du point de savoir si l'application (Z) cible est enregistrée comme application sécurisée ou est une application non sécurisée
- si l'application (Z) cible est une application sécurisée : accès de l'application (Z) cible au clipboard (C) pour accéder au jeu (BDₐ) de données image anonymisé associé à la clé (key) détectée et mise sur l'application (Z) cible
- si l'application (Z) cible est une application sécurisée : acheminement de l'instruction d'insertion de l'application (Z) cible au trader (TR), le trader (TR) accédant, par la clé (key) détectée, au module (S) de mémoire, afin d'accéder a jeu (BD_{key}) de données image chiffré associé à la clé respective et le déchiffrer par la clé (key) et mettre à disposition le jeu (BD) de données d'image déchiffré sur l'application (Z) cible.

2. Procédé suivant la revendication 1, dans lequel on associe un jeton (T) comme caractéristique d'identification respectivement à une instance d'un jeu (BD, BDₐ, BD_{key}) de données d'image, de manière à ce que l'on puisse accumuler même plusieurs jeux (BD) de données d'image ou des parties de ceux-ci dans le clipboard (C) et/ou dans le module (S) de mémoire et exécuter ainsi également une instruction multi-paste.

3. Procédé suivant l'une des revendications précédentes, dans lequel on utilise la clé (key) en même temps pour deux fonctions différentes, à savoir pour chiffrer le jeu (BD) de données d'image et pour identifier un jeu (BD) de données d'image.

4. Procédé suivant l'une des revendications précédentes, dans lequel on mémorise sur le module (S) de mémoire une instance d'association comprenant au moins la clé (key), le jeu (BDₐ) de données d'image anonymisé associé à la clé (key) respective et le jeu (BD_{key}) de données d'image chiffré associé à la clé (key) respective.

5. Procédé suivant l'une des revendications précédentes, dans lequel tous les appareils (G) sur lesquels l'utilisateur est entré reçoivent un message (N) que des jeux (BD) de données d'image ont été mémorisés pour l'utilisateur dans le clipboard (C) et/ou dans le module (S) de mémoire.

6. Procédé suivant l'une des revendications précédentes, dans lequel, pour respectivement un appareil (G), sur lequel l'application (Q) source et/ou l'application (Z) cible sont mises en oeuvre, on installe un trader (Tr).

7. Produit de programme d'ordinateur ayant un code de programme d'ordinateur pour effectuer le procédé suivant l'une des revendications précédentes, dans lequel le code de programme d'ordinateur est chargé dans une mémoire d'un ordinateur ou d'un appareil (G) informatique ou peut l'être lorsque le code de programme d'ordinateur est réalisé sur l'ordinateur.

8. Système de transmission pour des jeux de données d'image médicales critiques du point de vue de la sécurité avec Portected Health Information,
de parties PHI d'une application (Q) source à une application (Z) cible, comprenant :
- une application (Q) source, dans laquelle au moins une instruction de copie d'au moins un jeu (BD) de données d'image est détectée
- une application (Z) cible, dans laquelle au moins une instruction d'insertion est détectée
- une infrastructure PKI, qui, pour chaque utilisateur, met à disposition une clé (key) spécifique à l'utilisateur
- un module (Tr) de trader, sur lequel est produit, pour chaque jeu (BD) de données d'image, un jeu (BDₐ) de données d'image anonymisé et un jeu (BD_{key}) de données d'image chiffré
- un module (S) de mémoire, dans lequel au moins le jeu (BD_{key}) de données d'image chiffré est mémorisé
- un clipboard (C), dans lequel au moins le jeu (BDₐ) de données d'image anonymisé est mémorisé
- dans lequel le système de transmission peut desservir en même temps des applications cibles reconnues comme sécurisées et reconnues comme non sécurisées, dans lequel, pour une application (Z) cible non sécurisée, celle-ci accède, par la clé (key), au clipboard (C) pour détecter le jeu (BDₐ) de données d'image anonymisé associé à la clé et le représenter sur l'application (Z) cible et dans lequel, pour une application (Z) cible non sécurisée, le module (Tr) de trader accède par la clé (key) au module (S) de mémorisation pour détecter le jeu (BD_{key}) de données d'image chiffré et le déchiffrer par la clé et insérer ainsi le jeu (BD) de données d'image déchiffré sur l'application (Z) cible.
